# EUROPEAN PATENT SPECIFICATION

(11) **EP 1 542 761 B1**
(45) Date of publication and mention of the grant of the patent: **05.12.2007**
(21) Application number: 03725858.9
(22) Date of filing: 02.06.2003
(51) Int. Cl.: A61F 2/90

(54) **A CYLINDRICAL STENT**
ZYLINDRISCHER STENT
IMPLANT ENDOVASCULAIRE CYLINDRIQUE

(43) Date of publication of application: 22.06.2005
(73) Proprietor: S & G Biotech, Inc., Seoul 143-853 (KR)
(72) Inventor: KANG, Sung-Gwon, Yongin-Si, 449-845 Gyeonggi-Do (KR); KIM, Eun-Sang, Nowom-Gu, Seoul (KR)
(74) Representative: Wagner, Bernhard Peter
(86) International application number: PCT/KR2003/001077
(87) International publication number: WO 2004/105852

(56) References cited:
- EP-A- 1 308 138
- WO-A1-94/20044
- KR-A- 2001 084 836
- KR-B1- 267 019
- KR-B1- 269 572
- KR-B1- 319 663
- US-A- 5 800 515
- US-A- 5 897 589
- US-A1- 2002 040 236

## Description

### TECHNICAL FIELD

The present invention relates to a medical appliance for lumens, and particularly, to a self-expandable medical appliance (hereinafter, referring to a 'stent') for expanding the lumens of a blood vessel or esophagus in case these are constricted. More particularly, the present invention relates to a stent which can be contracted to a minimum size, is superior in expansibility, is easily bent and is very superior in flexibility.

Typically, the lumens in a human body may be constricted by diseases occurring in the body and thus their functions are degraded, more seriously, disabled. Such situation arises, for example, when the esophagus is constricted due to esophageal cancer, when the blood does not flow smoothly due to arteriosclerosis, or when a track for flowing bile produced from a liver is constricted.

In such a condition that the lumen is constricted, food, blood or bile cannot flow smoothly. Thus, expanding the constricted lumen for maintaining an open passageway is needed in such cases, and for this purpose, for example, a stent is inserted into the lumen as a method of expanding the constricted passageway and maintaining an open passage way.

Such a stent is widely used which has an overall shape of a cylindrical structural body, is self-elastic to thus be contracted by an external force, and is self-expanding when the external force is removed.

The above-mentioned self-expandable stent requires many properties, i.e., expansibility for expanding the lumen, flexibility for flexibly adapting itself while maintaining a bent shape of the lumen at a bent portion of the lumen, and contractibility for contracting into a predetermined diameter, in order to perform its functions properly.

Among the properties of the stent, if it is short of expansibility, it is easily moved from a first stent section of the lumen, if it is short of flexibility, it is reduced in adaptability to a bent portion of the lumen when the stent expands from the bent portion of the lumen, and if it is short of contractibility, the size of an insertion device for inserting the stent is increased.

### BACKGROUND ART

Therefore, a plurality of stents having the above-mentioned functions has been developed. The conventional stents are mainly divided into spiral type stents and zigzag type stents.

The representative ones of the spiral type stents includes a stent which is formed in a cylindrical shape on the whole by spirally wrapping filaments having a unit length along a cylindrical wall face, reciprocating them between opposite ends in a zigzag pattern and crossing them each other, thereby producing a mesh. The stent of this type is superior in flexibility because it can be contracted into a small diameter, while having a drawback that it is largely decreased in expansibility due to the flexibility.

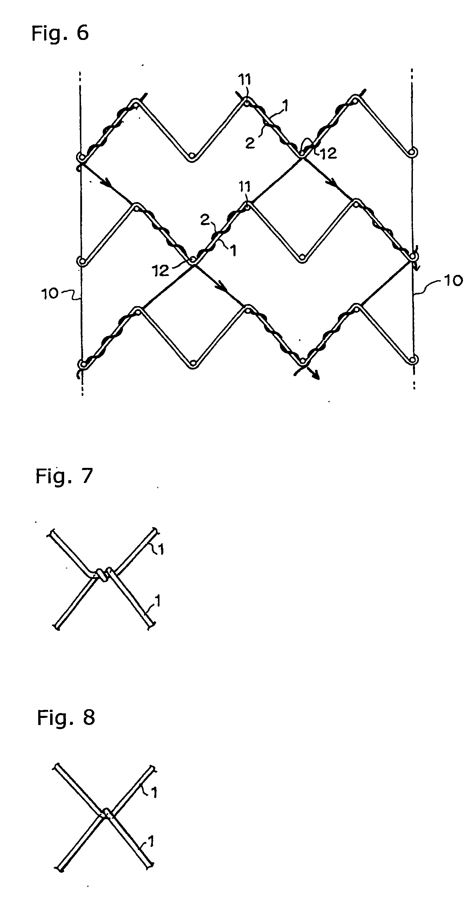

Korean Patent Laid-Open No. 1998-67399 discloses a stent which is formed of a filament having a plurality of turns of a zigzag configuration consisting of a plurality of straight portions and a plurality of peak and valley portions connecting the straight portions by a plurality of bending portions, and wherein the valley portion of a certain turn of the stent is twisted and connected with the peak portion of an adjacent turn of the stent as shown in Figs. 7 and 8.

Although the conventional stents are superior in the expansibility for expanding the constricted lumen, they are disadvantageous in that they are poor in flexibility, are difficult to be contracted to a small size and cannot maintain a bent shape at a bent portion of the lumen as its.

US 5,897,589 is concerned with an endoluminal medical implant and discloses a cylindrical stand being formed of a wire and having a longitudinal axis. The stand comprises a plurality of basic terms being arranged with no crossing points to each other. The basic terms of a zigzag configuration consists of a plurality of peak portions and a plurality of valley portions connected by straight portions along the circumferential surface, and are connected to each other by a plurality of connecting terms by winding the straight portions of the overlapped basic terms in a coiled shape.

### DISCLOSURE OF INVENTION

The invention is defined in appended claim 1. Dependent claims 2 and 3 define preferred embodiments of the invention.

Accordingly, it is an object of the present invention to provide a stent which can be contracted to a minimum size because it has an open area (3) In a unit cell, maintain a cylindrical shape well for smoothly flowing blood or the like at a bent portion of a lumen because of its superior expansibility and flexibility, is bent according to a bent shape of the lumen and is closely contacted with an inner wall of the lumen.

To achieve the above object, there is provided a cylindrical stent being formed of a wire and having a longitudinal axis according to the present invention, comprising: a plurality of basic turns being arranged with no crossing points to each other at an interval twice the height (h) of the basic turns along the longitudinal direction of a cylindrical body, the basic turns (1) of a zigzag configuration consisting of a plurality of peak portions (1c) and a plurality of valley portions (1b) connected by straight portions (1a) along the circumferential surface; and a plurality of connecting turns (2) connecting the plurality of basic turns each other by winding the straight portions (1a) of the overlapped basic turns in a coil shape and being arranged at an interval twice the distance (d) of the straight portions of the basic turns in a diagonal direction to the left and right sides, the basic turns being overlapped with the connecting turns as the connecting turns are arranged in a diagonal direction to the left and right sides of the basic turns.

### BRIEF DESCRIPTION OF THE DRAWINGS

These and other features, aspects, and advantages of preferred embodiments of the present invention will be more fully described in the following detailed description, when taken in conjunction with accompanying drawings. In the drawings:
Fig. 1 is a front perspective view of a stent according to the present invention;
Fig. 2 is an enlarged view showing a unit cell of the stent according to the present invention;
Fig. 3 is an enlarged view showing a basic turn of the stent according to the present invention;
Figs. 4 to 6 are views for explaining a method for making a stent according to the present invention;
Figs. 7 to 8 are views showing a connecting state of basic turns in a conventional stent.

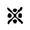 Explanation of basic numerals for main parts of drawings.

1: basic turn 2: connecting turn 3: open area
1a: straight portion of basic turn
1b: valley portion of basic turn
1c: peak portion of basic turn
d: distance between straight portions of basic turn
h: height of basic turn
H: interval between basic turns
D: interval between connecting turns
10: jig for manufacture of stent
11: first basic pin 12: second basic pin

### BEST MODES FOR CARRYING OUT THE INVENTION

Hereinafter, the present invention will be described in detail with basic to the accompanying drawings.

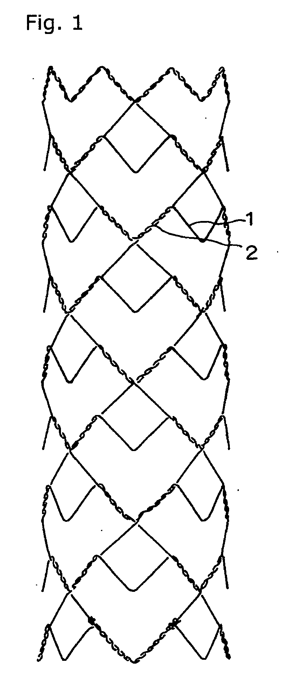

Referring to Fig. 1, the cylindrical stent of the invention includes a plurality of basic turns 1 which is made of a wire having a unit length and has a zigzag configuration, the wire having a plurality of bending points along the circumferential face.

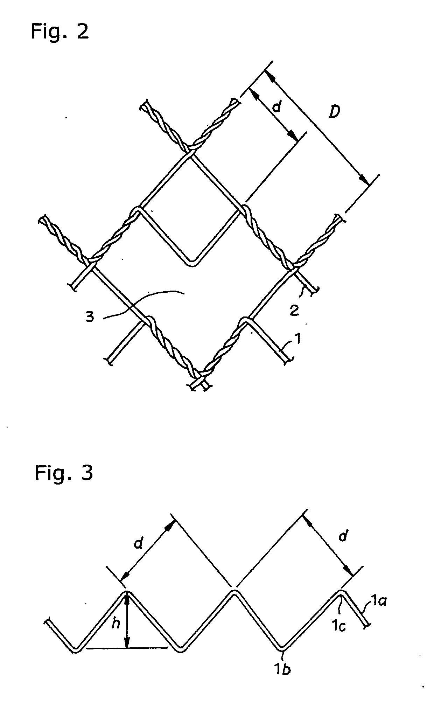

The basic turns have a plurality of peak portions 1c and a plurality of valley portions 1b as shown in Fig. 3. The peak portions 1c and the valley portions 1b are connected by straight portions 1a slanted to the left and right sides. The basic turns 1 are arranged with no crossing points to each other at an interval twice the height (h) of the basic turns along the longitudinal direction of a cylindrical body.

Meanwhile, as shown in Fig. 1, the cylindrical stent of the present invention includes a plurality of connecting turns 2 which connects the plurality of basic turns by winding the straight portions 1a of the overlapped basic turns in a coil shape, the basic turns being overlapped with the connecting turns as the connecting turns are arranged in a diagonal directions to the left and right sides of the basic turns. The connecting turns 2 are arranged at an interval twice the distance (d) between the straight portions 1a of the basic turns.

By the basic turns 1 and connecting turns 2 above-described, the stent of the invention forms a unit cell as shown in Fig. 2.

The unit cell of Fig. 2 has an area where the connecting turns 2 connecting the straight turns 1a of adjacent basic turns are not arranged, that is, an open area, to thus improve the flexibility of the stent greatly. This can contract the stent to a smaller size, and the stent can keep its original shape even at a bent portion of a lumen, thereby allowing the flow of blood to be performed smoothly.

In addition, in the present invention, the connecting turns 2 are wound about the straight portions 1a of the overlapped basic turns in a coil shape, so the expansibility of the stent is also superior. The number of times the connecting turns 2 are wound about the straight portions 1a of the basic turns is preferably 2 to 10. The wire constituting the basic turns 1 and the connecting turns 2 is preferably made of a shape memory alloy such as nitinol.

An example of a method for making a cylindrical stent of the present invention will now be described.

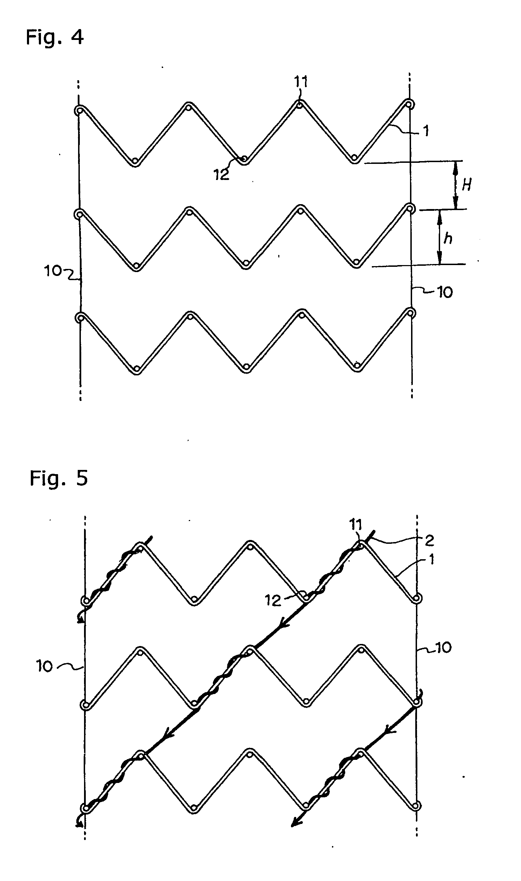

Firstly, as shown in Fig. 4, the wire is revolved once in a zigzag manner along a first basic pin 11 and a second basic pin 12 of a jig 10 for the manufacture of a stent to thus form a single basic turn 1. At a position spaced apart from the thus-formed basic turn in a downward direction of the jig 10 at an interval twice the height (h) of the basic turn, another basic turn 1 is formed in the same manner. By repeating this work, a plurality of basic turns is formed which is arranged along the longitudinal direction of a cylindrical body without no crossing points to each other.

Next, as shown in Figs. 5 and 6, a plurality of connecting turns 2 connecting the straight portions 1a of the basic turns in a direction diagonal to the left and right sides. At this time, the interval between the connecting turns 2 is adjusted to be twice the distance (d) between the straight portions of the basic turns, and the connecting turns 2 are wound around the straight portions 1a of the basic turns overlapped with the connecting turns 2.

Once the formation of the connecting turns 2 is over, the wire is positioned at the first basic pin 11 of the jig 10. At this time, if the wire is knotted, the thinning work of the stent is completed.

In the present invention, the knot of the connecting turns 2 serves as a fixing wire for fixing the stent into a lumen, and thus the manufacture and attachment of a fixing wire separately can be minimized.

Next, the resulting stent is heated for 10 to 20 minutes in a heating oven set to 400°C to 600°C along with the jig 10 for the manufacture of the stent, then is annealed for 30 minutes to 4 hours at a room temperature, then is separated from the jig 10, and then is finished, thereby completing the manufacture of the stent.

The present invention does not specially limit the condition of heating and annealing for the manufacture of the stent.

### INDUSTRIAL APPLICABILITY

The cylindrical stent of the present invention is very superior in flexibility and expansibility since it has an open area in a unit cell. As a result, it can be contracted to a minimum size, keep its original shape at a bent portion of a lumen and is superior in adhesiveness to the bent portion of the lumen. Moreover, the present invention can minimize a number of fixing wires manufactured separately.

## Claims

1. A cylindrical stent being formed of a wire and having a longitudinal axis, comprising:
- a plurality of basic turns (1) being arranged with no crossing points to each other, the basic turns (1) of a zigzag configuration consisting of a plurality of peak portions (1c) and a plurality of valley portions (1b) connected by straight portions (1a) along the circumferential surface; and
- a plurality of connecting turns (2) connecting the plurality of basic turns (1) to each other by winding the straight portions (1a) of the overlapped basic turns in a coil shape;
**characterized in that**
- the basic turns (1) being arranged at an interval twice the height (h) of the basic turns along the longitudinal direction of a cylindrical body and
- the connecting turns (2) being arranged at an interval (D) twice the distance (d) of the straight portions (1a) of the basic turns (1) in a diagonal direction to the left and right sides.

2. The cylindrical stent of claim 1, wherein the wire is made of a shape memory alloy such as nitinol.

3. The cylindrical stent of claim 1, wherein the connecting turns (2) are wound about the straight portions (1a) of the overlapped basic turns 2 to 10 times.

## Patentansprüche

1. Zylindrischer Stent, der aus einem Draht geformt ist und eine Längsachse besitzt und umfasst:
- mehrere Basiswindungen (1), die ohne Kreuzungspunkte miteinander angeordnet sind, wobei die Basiswindungen (1) eine Zickzack-Konfiguration besitzen, die aus mehreren Scheitelabschnitten (1c) und aus mehreren Talabschnitten (1b), die durch geradlinige Abschnitte (1a) längs der Umfangsoberfläche verbunden sind, besteht; und
- mehrere Verbindungswindungen (2), die die mehreren Basiswindungen (1) miteinander verbinden, indem sie die geradlinigen Abschnitte (1a) der überlappten Basiswindungen in eine Spulenform wickeln;
**dadurch gekennzeichnet, dass**
- die Basiswindungen (1) in einem Intervall angeordnet sind, das gleich der doppelten Höhe (h) der Basiswindungen in der Längsrichtung eines zylindrischen Körpers ist, und
- die Verbindungswindungen (2) in einem Intervall (D) angeordnet sind, das gleich dem doppelten Abstand (d) der geradlinigen Abschnitte (1a) der Basiswindungen (1) in einer diagonalen Richtung nach links und nach rechts ist.

2. Zylindrischer Stent nach Anspruch 1, wobei der Draht aus einer Legierung mit Formerinnerungsvermögen wie etwa Nitinol hergestellt ist.

3. Zylindrischer Stent nach Anspruch 1, wobei die Verbindungswindungen (2) 2- bis 10-mal um die geradlinigen Abschnitte (1a) der überlappten Basiswindungen gewunden sind.

## Revendications

1. Implant endovasculaire cylindrique formé d'un fil et présentant un axe longitudinal, comprenant :
- une pluralité de spires de base (1) étant agencées sans aucun point de croisement les uns avec les autres, les spires de base (1) présentant une configuration en zigzag constituée d'une pluralité de parties de sommet (1c) et d'une pluralité de parties de creux (1b) raccordées par des parties rectilignes (1a) le long de la surface circonférentielle ; et
- une pluralité de spires de raccordement (2) raccordant la pluralité de spires de base (1) les unes aux autres en enroulant les parties rectilignes (1a) des spires de base se chevauchant selon une forme de bobine ;
**caractérisé en ce que**
- les spires de base (1) sont agencées suivant un intervalle mesurant deux fois la hauteur (h) des spires de base le long de la direction longitudinale d'un corps cylindrique et
- les spires de raccordement (2) sont agencées suivant un intervalle (D) mesurant deux fois la distance (d) des parties rectilignes (1a) des spires de base (1) dans une direction diagonale par rapport aux côtés gauche et droit.

2. Implant endovasculaire cylindrique selon la revendication 1, dans lequel le fil est fabriqué à partir d'un alliage à mémoire de forme tel que le nitinol.

3. Implant endovasculaire cylindrique selon la revendication 1, dans lequel les spires de raccordement (2) sont enroulées autour des parties rectilignes (1a) des spires de base se chevauchant 2 à 10 fois.
